# FASCICULE DE BREVET EUROPEEN

(11) **EP 0 438 203 B1**
(45) Date de publication et mention de la délivrance du brevet: **06.11.1996**
(21) Numéro de dépôt: 91200556.8
(22) Date de dépôt: 03.04.1987
(51) Int. Cl.: C07H 19/067, C07H 19/073, C07H 21/00

(54) **Dérivés de nucléosides et leur utilisation pour la synthèse d'oligonucléotides**
Nucleosidderivate und ihre Verwendung in der Oligonucleotid-Synthese
Nucleoside derivatives and their use in oligonucleotide synthesis

(30) Priorité: 08.04.1986 FR 8604990
(43) Date de publication de la demande: 24.07.1991
(62) Demande divisionnaire de: 87400739.6
(73) Titulaire: COMMISSARIAT A L'ENERGIE ATOMIQUE, 75015 Paris (FR)
(72) Inventeur: Molko, Didier, F-38120 Tullins (FR); Schulhof, Jean-Claude, F-38330 Saint Ismier (FR); Teoule, Robert, F-38000 Grenoble (FR)
(74) Mandataire: Des Termes, Monique

(56) Documents cités:
- EP-A- 0 090 789

## Description

La présente invention a pour objet de nouveaux dérivés de nucléosides et leur utilisation pour la synthèse d'oligonucléotides.

De façon plus précise, elle concerne des dérivés de nucléosides formés à partir de cytosine, qui sont utilisables en particulier pour la synthèse d'oligonucléotides.

La synthèse d'oligonucléotides consiste à lier entre eux des nucléosides par un groupement phosphate pour former une chaîne d'ADN (acide désoxyribonucléique) ou d'ARN (acide ribonucléique). Dans cet assemblage, les groupements phosphates internucléotidiques relient toujours la fonction hydroxyle en position 3' d'un nucléoside à la fonction hydroxyle en position 5' d'un autre nucléoside. Ainsi, lors de la réaction de synthèse, seules les extrémités 3' et 5' des nucléosides sont sollicitées et la base nucléique (purique ou pyrimidique) utilisée ne doit pas intervenir au cours de cet assemblage.

Lorsque ces bases comprennent des groupements NH₂ exocycliques, il est nécessaire de protéger ces groupements lors de la synthèse des oligonucléotides car ceux-ci sont trop réactifs et risquent d'interférer avec les réactions de synthèse.

Cette protection des groupements NH₂ exocycliques doit répondre à certaines caractéristiques :
- elle doit être sélective et facile à réaliser,
- elle ne doit pas induire de modifications de réactivité des autres sites du nucléoside, et être stable pendant toutes les étapes de la synthèse oligonucléotidique, et
- elle doit pouvoir être éliminée ensuite dans des conditions douces sans détruire l'oligonucléotide qui vient d'être synthétisé.

Les groupements NH₂ exocycliques des nucléosides ont le plus souvent été protégés sous la forme d'amides, par exemple au moyen de groupes benzoyle ou anisoyle dans le cas de l'adénine et de la cytosine comme il est décrit par H. SCHALLER et al dans J. Amer. Chem. Soc. (1963), vol. 85, p. 3821-3827, et au moyen du groupe isobutyryle dans le cas de la guanine comme il est décrit par H.BÜCHI et H. KHORANA dans J. Mol. Biol. (1972), vol. 72, p. 251-288.

Ces groupes protecteurs peuvent être éliminés en fin de synthèse par action de l'ammoniaque à 28%, pendant 17 h, à une température de 60°C, comme il est préconisé. Cependant la RMN du proton montre que dans ces conditions on n'élimine pas tous les groupements isobutyryle de la guanine ; il est donc préférable de porter le temps de réaction à 72 heures, toujours à une température de 60°C.

Ce mode d'élimination des groupes protecteurs constitue un inconvénient car les conditions mises en oeuvre ne sont pas suffisamment douces pour pouvoir être utilisées avec des nucléosides modifiés, peu stables en milieu alcalin, comme c'est le cas par exemple de la dihydro-5,6 thymidine.

Aussi, des recherches ont été entreprises sur la possibilité d'utiliser d'autres groupements acyle plus faciles à éliminer, utilisables en particulier pour la synthèse d'oligonucléotides à partir de nucléosides labiles par la méthodologie de synthèse sur support qui consiste à fixer le premier nucléoside de la chaîne sur un support, généralement en silice, à effectuer ensuite successivement des cycles de condensation pour fixer sur le premier nucléoside les autres nucléosides dans l'ordre voulu. L'utilisation de groupements acyle plus faciles à éliminer permet par ailleurs d'obtenir un meilleur rendement de déprotection. Ce point est très important car la présence de bases incomplètement déprotégées présente un inconvénient pour l'utilisation des produits obtenus.

Des résultats d'études sur la cinétique de dissociation de groupes protecteurs aliphatiques du 2'-désoxycytidine-5'-phosphate, dans NaOH, à des pH allant de 11 à 14, ont été publiés par Seliger et al dans Chem. Ber., 111, p. 3732-3739, 1978. Ils ont montré que le groupe propionyle est le plus labile, et que les groupes acétyle, isobutyryle et n-butyryle sont très peu stables à l'hydrolyse dans NaOH.

Köster et al dans Tetrahedron, vol 37, 1981, p. 363-369, ont étudié d'autres groupes protecteurs pour la synthèse oligonucléotidique en vue de choisir des groupes présentant une meilleure stabilité à l'hydrolyse que ceux décrits par Seliger.

Ainsi, aucune de ces études n'a abouti au choix d'un groupe présentant toutes les propriétés requises.

La présente invention a précisément pour objet de nouveaux dérivés de nucléosides comportant un groupement protecteur de type acyle, susceptible d'être éliminé facilement.

Les dérivés de nucléosides de l'invention répondent à la formule : 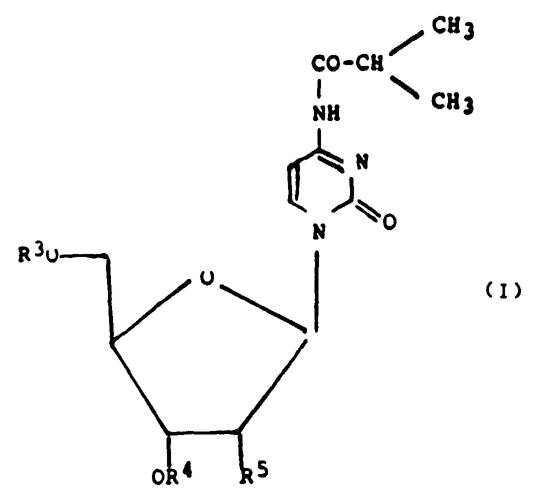 dans laquelle
- R³ représente un atome d'hydrogène, un radical labile en milieu acide utilisable pour la synthèse oligonucléotidique ou le radical de formule : 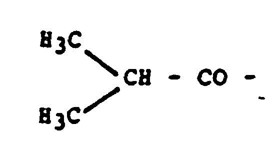
- R⁴ représente un atome d'hydrogène, un radical phosphoré utilisable pour la synthèse oligonucléotidique ou le radical : 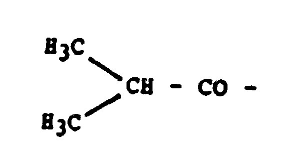 et
- R⁵ représent un atome d'hydrogène, le radical OH ou un radical OH protégé.

A titre d'exemple, les radicaux labiles en milieu acide susceptibles d'être utilisés pour former R³ dans le composé de formule (I) sont en particulier des radicaux utilisables en synthèse oligonucléotidique tels que :
- les radicaux trityle répondant à la formule : 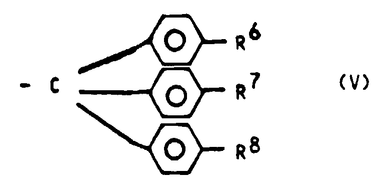 dans laquelle R⁶, R⁷ et R⁸ qui peuvent être identiques ou différents, représentent un atome d'hydrogène, un radical alkyle ou un radical alkoxy, par exemple le radical monométhoxytrityle ou le radical trityle de formule (V) dans laquelle R⁶ et R⁷ représentent le radical méthoxy et R⁸ représente un atome d'hydrogène ;
- les radicaux pixyle et les radicaux phényl-9 xanthènyle.

A titre d'exemple, les radicaux phosphorés susceptibles d'être utilisés pour former R⁴ dans le composé de formule (I) sont également des radicaux utilisables en synthèse oligonucléotidique tels que le radical de formule : 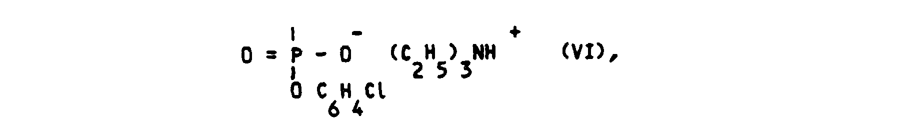 le radical de formule : 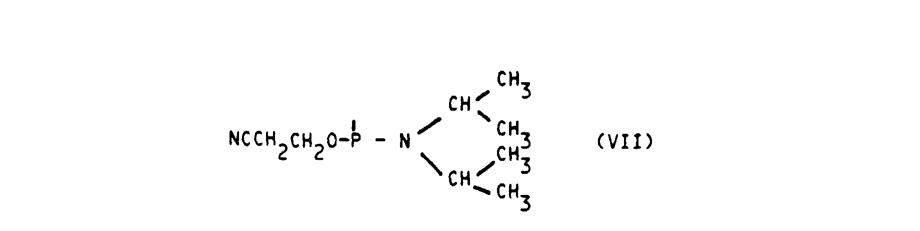 le radical phosphonate de formule :

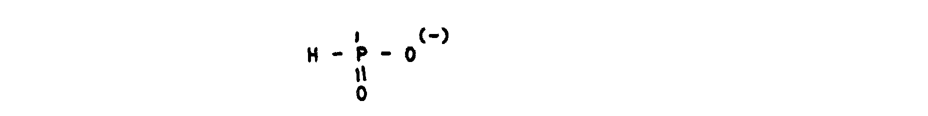

Selon l'invention, lorsque R⁵ représente le radical OH protégé, on utilise comme groupe protecteur de OH les groupes habituellement utilisés dans la synthèse des ribonucléotides.

Les dérivés de nucléosides de l'invention sont ainsi les produits de l'union 1°) d'une base formée par la cytosine et 2°) du ribose ou du désoxyribose, les nucléosides étant modifiés au moins sur le groupement NH₂ exocyclique de leur base par un groupement 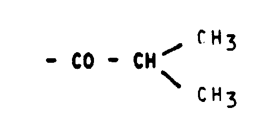 Ils peuvent également être modifiés par ce même groupement sur les positions 3' et 5' du désoxyribose ou 2', 3' et 5' du ribose, ou les positions 3' et 5' du ribose ou du désoxyribose peuvent être modifiées par d'autres groupements qui sont les groupements labiles R³ pour la position 5' et le groupement phosphoré R⁴ sur la position 3' du ribose ou du désoxyribose.

Le groupement acyle de formule 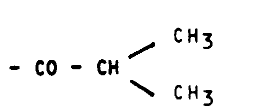 utilisé dans l'invention est particulièrement intéressant pour la synthèse des nucléotides car il peut être éliminé facilement en fin d'opération, par exemple par traitement à l' ammoniaque pendant 2 à 8 h selon le groupement utilisé, à la température ambiante, ce qui permet de libérer simultanément le polynucléotide du support sur lequel il a été synthétisé, lorsqu'on utilise la méthode de synthèse sur support.

L'utilisation du groupe protecteur mentionné ci-dessus permet d'obtenir une réduction du temps de déprotection des oligonucléotides obtenus par assemblage des nucléosides de l'invention, puisque ce temps peut être seulement de 2 à 8 h selon le groupement utilisé, au lieu des 17 à 72 h nécessaires antérieurement. On peut par ailleurs opérer dans des conditions réactionnelles plus douces puisqu'on réalise la déprotection à température ambiante alors qu'il était nécessaire auparavant de chauffer à 60°C. De plus, l'utilisation de ces groupes protecteurs éliminables plus facilement permet d'incorporer lors de la synthèse des oligonucléotides des bases nucléiques modifiées sensibles à des conditions alcalines plus violentes, par exemple de synthétiser des fragments d'ADN porteurs de ligands sensibles à des anticorps.

Bien que l'invention s'applique aux nucléosides dérivés du ribose et aux nucléosides dérivés du désoxyribose, on l'utilise de préférence pour les nucléosides dérivés du désoxyribose, c'est-à-dire aux dérivés de formule (I) dans laquelle R⁵ est un atome d'hydrogène.

Les dérivés de nucléosides de l'invention peuvent être préparés par des procédés classiques, analogues à ceux utilisés pour fixer le groupement benzoyle sur les nucléosides à base de cytosine. Dans ces procédés, on part du nucléoside de la cytosine que l'on fait réagir avec le chlorure d'acide de formule : 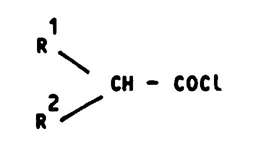 ou l'anhydride d'acide de formule :

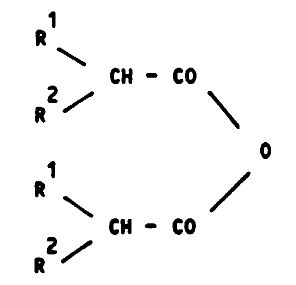

Lors de cette réaction, l'anhydride ou le chlorure d'acide réagit également avec les groupements hydroxyle en position 3' et 5' du ribose ou du désoxyribose et l'on obtient ainsi le dérivé du nucléoside triprotégé soit le dérivé de formule (I) dans lequel R³ et R⁴ représentent tous deux le radical :

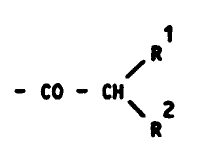

On peut toutefois éliminer ces radicaux en position 3' et 5' par hydrolyse sélective, ce qui permet d'obtenir les dérivés de nucléosides de formule (I) dans laquelle R³ et R⁴ représentent des atomes d'hydrogène.

On peut préparer les dérivés de nucléosides de formule (I) dans laquelle R³ représente un radical trityle de formule (V), par exemple le groupement diméthoxytrityle, et R⁴ représente un atome d'hydrogène en faisant réagir les dérivés des nucléosides obtenus précédemment avec le chlorure de trityle correspondant dans un solvant approprié.

Les dérivés de nucléosides répondant à la formule (I) dans laquelle R³ représente un groupement trityle et R⁴ représente soit le radical de formule (VI), soit le radical de formule (VII), soit un radical de formule : 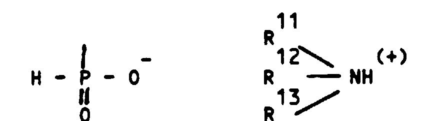 dans laquelle R¹¹, R¹² et R¹³ qui peuvent être identiques ou différents, sont des radicaux alkyle, par exemple éthyle, peuvent être préparés par des procédés classiques à partir des dérivés de nucléosides de formule (I) dans laquelle R³ représente un radical trityle et R⁴ représente un atome d'hydrogène.

Dans le cas où R⁴ représente, par exemple, le radical de formule (VI), on fait réagir ce dérivé de nucléoside avec du bistriazolidate de chloro-4 phénylphosphoryle dans un solvant approprié. Le bistriazolidate de chloro-4 phénylphosphoryle peut être préparé par addition de dichlorophosphate de chloro-4 phényle à une suspension de triazole et de triéthylamine dans du dioxanne.

Lorsque R⁴ représente, par exemple, le radical de formule (VII), on peut faire réagir le dérivé de nucléoside avec du β-cyanoéthyl-monochloro N,N-diisopropyl amino phosphoramidite dans un solvant approprié en présence de diisopropyléthylamine.

Lorsque R⁴ représente, par exemple, le radical de formule : 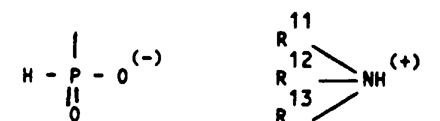 dans laquelle R¹¹, R¹² et R¹³ qui peuvent être identiques ou différents, sont des radicaux alkyle, on peut faire réagir le dérivé de nucléoside avec de la chloro-2 benzo(5,6-a) [dioxo-1,3 phosphor-2 inone-4] puis avec un sel de trialkyl ammonium tel que l'acétate de triéthyl ammonium.

Les dérivés de nucléosides obtenus par ces trois méthodes peuvent être utilisés pour la synthèse d'ologonucléotides soit par synthèse phosphotriester dans le cas ou R⁴ est le radical de formule (VI), soit par synthèse phosphoramidite dans le cas où R⁴ est le radical de formule (VII), soit par synthèse H-phosphonate dans le cas où R⁴ est le radical de formule : 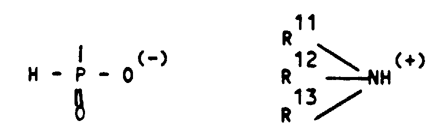 en utilisant également pour l'assemblage des chaîne oligonucléotidiques d'autres nucléosides, par exemple ceux correspondant à la thymidine et à la désoxy-2' uridine, ou encore des nucléosides comportant des bases labiles en milieu alcalin ou d'autres nucléosides labiles en milieu alcalin.

Le procédé, selon l'invention, de synthèse d'oligonucléotides, comprend :
1° au moins un cycle de condensation dans lequel on condense sur un dérivé de nucléoside ou sur un oligonucléotide un dérivé de nucléoside de formule : 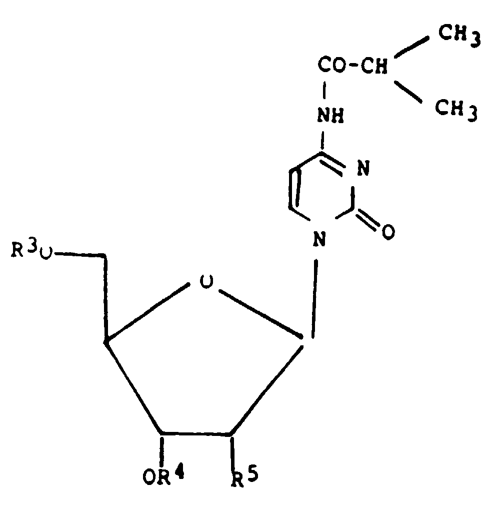 dans laquelle
   - R³ représente un radical labile en milieu acide pour synthèse oligonucléotidique,
   - R⁴ représente un radical phosphoré pour synthèse oligonucléotidique, et
   - R⁵ représente un atome d'hydrogène ; et
2°) une étape d'élimination du ou des groupes protecteurs de formule :

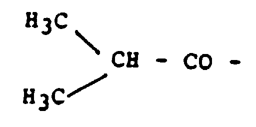

La synthèse oligonucléotidique peut être effectuée soit par des méthodes en solution, soit par des méthodes de synthèse sur support. On utitise de préférence les méthodes de synthèse sur support car celles-ci sont mieux adaptées à l'utilisation de nucléosides plus labiles, sans qu'il y ait une perte de rendement au cours de l'assemblage.

Les nucléosides de l'invention peuvent donc trouver des applications intéressantes en tant que produits de base destinés à la synthèse de fragments d'ADN ou d'ARN. Ils peuvent également convenir pour incorporer dans des otigonuctéotides de synthèse des bases modifiées fragiles, ce qui peut concerner en particulier les produits de radiolyse gamma de l'ADN ainsi que les produits de photolyse.

Les nucléosides de l'invention peuvent permettre également l'accès à de nouvelles molécules à activité antivirale et à de nouvelles sondes d'ADN.

Les exemples suivants de préparation et d'utilisations des nucléosides conformes à l'invention sont donnés bien entendu à titre non limitatif pour illustrer l'invention. Les composés 1 à 4, 7 à 10, 12, 13 et 15 à 18 ne font pas partie de l'invention.

### EXEMPLE 1 : Préparation de (N₂-phénoxyacétyl)-désoxy-2' guanosine (composé n°1)

On sèche 1080 mg (4 mmol) de désoxy-2' guanosine par additions et évaporations successives de pyridine anhydre, puis on les met en suspension dans 20 ml de pyridine anhydre et on introduit la suspension dans un ballon. On refroidit le ballon au bain de glace et on y ajoute lentement 6 équivalents 14,25 g ; 24 mmol) de chlorure de phénoxyacétyte à 0°C. On laisse ensuite la réaction se poursuivre à la température ambiante pendant 90 min. Un précipité blanc de chlorure de pyridinium apparaît dans le milieu réactionnel tandis que ceLui-ci prend une coloration orangée à brune. On obtient ainsi le dérivé triprotégé du nucléoside de départ. On détruit alors l'excès de chlorure d'acide à 0°C par 2 ml d'eau bidistillée, ce qui solubilise le milieu réactionnel puis on le dilue par 100 ml de chloroforme. On lave 4 fois la phase chloroformique par 50 ml de solution aqueuse à 5% de bicarbonate de sodium pour éliminer l'acide phénoxyacétique formé. On sèche alors la phase chloroformique sur du sulfate de sodium, on évapore le solvant et on obtient ainsi un résidu orange. On dissout celui-ci dans 100 ml de pyridine refroidis à 0°C, puis on ajoute 100 ml de soude 0,2 N à 0°C. On effectue ainsi l'hydrolyse sélective des positions 3' et 5' en 20 min. On neutralise alors le milieu au moyen de la résine échangeuse de cations, Dowex 50W-X8 d'une granulométrie de 100 à 200 mesh (0,074 à 0,149 mm), sous forme pyridinium. Après filtration et rinçage de la résine, on évapore à sec le filtrat.

On isole ensuite la N₂-phénoxyacétyl-désoxy-2' guanosine formée par chromatographie sur une colonne de silice (3 cm de diamètre, 15 cm de hauteur) que l'on élue par un gradient de chloroforme-méthanol. L'évaporation des fractions contenant le produit recherché permet de recueillir 250 mg de N₂-phénoxyacétyl-désoxy-2' guanosine, ce qui correspond à un rendement de 15%.

On contrôle l'identité et la pureté du produit obtenu par résonance magnétique nucléaire à 250 MHz, par chromatographie en couche mince et par spectrométrie de masse. On obtient les résultats suivants :
- R_{F} = 0,36 avec le mélange de migration chloroforme-méthanol (80/20 en vol),
- (M + H) pic moléculaire (m/e : 402 - 13%) ; guanine phénoxyacétylée (m/e : 286 - 51%).

### EXEMPLE 2 : Préparation de[N₂-(chloro-2 phénoxy)acétyl]-désoxy-2' guanosine (composé n°2)

On sèche 1080 mg (4 mmol) de désoxy-2' guanosine comme dans l'exemple 1, puis on les met en suspension dans 20 ml de pyridine anhydre et on les introduit dans un ballon placé dans un bain d'eau glacée. On ajoute lentement à 0°C, 6 équivalents (5,1 g ; 24 mmol) de chlorure de (chloro-2(chloro-2-phénoxy)acétyle. On laisse la réaction se poursuivre à ta température ambiante pendant 150 min. Une coloration verte à marron apparaît dans le milieu réactionnel et on forme ainsi le dérivé triprotégé du nucléoside de départ.

On détruit l'excès de chlorure d'acide à 0°C par 2 ml d'eau distillée qui solubilisent le milieu réactionnel. On le dilue alors par 100 ml de chloroforme et on lave cette phase chloroformique par 4 fois 50 ml d'une solution aqueuse à 5% de bicarbonate de sodium pour éliminer l'acide chlorophénoxyacétique. On sèche la phase chloroformique sur du sulfate de sodium, on évapore le solvant et l'on obtient ainsi un résidu orangé. On dissout ce résidu dans 100 ml de pyridine, on place la solution obtenue dans un bain d'eau glacée et on lui ajoute 100 ml de soude 0,2 N, ce qui donne un mélange titrant 0,1N et permet d'hydrolyser sélectivement les positions 3' et 5' du nucléoside en 20 min. On neutralise alors le milieu par la résine échangeuse de cations Dowex 50W-X8 utilisée dans l'exemple 1 sous forme pyridinium. On filtre et on rince la résine, puis on évapore à sec le filtrat. On obtient ainsi la [N₂-(chloro-2 phénoxy)acétyl]-désoxy-2' guanosine qui est soluble uniquement dans la pyridine.

On la purifie par chromatographie sur colonne de silice en utilisant un gradient chloroforme-méthanol. On isole ainsi 220 mg du composé n°2, ce qui correspond à un rendement de 13%. On caractérise le composé par chromatographie en couche mince et par spectrométrie de masse.

Les résultats obtenus sont les suivants :
- R_{F} = 0,4 dans un mélange de migration chloroforme-méthanol (80/20 en volume),
- (M + H) : pic moléculaire (m/e : 436 - 17%) ; guanine chloro-2 phénoxyacétylée (m/e : 320 - 44%).

La pureté du produit est confirmée par analyse par résonance magnétique nucléaire à 250 MHz.

### EXEMPLE 3 : Préparation de (N₂-méthoxyacétyl)-désoxy-2' guanosine (composé n°3)

On sèche 5,4 g (20 mmol) de désoxyguanosine puis on les met en suspension dans 100 ml de pyridine anhydre. On refroidit à 0°C et on ajoute lentement 4,5 équivalents (10 g ; 90 mmol) de chlorure de méthoxyacétyle. On laisse la réaction se poursuivre à la température ambiante pendant 3 h pour former le dérivé triprotégé du nucléoside de départ.

On détruit l'excès de chlorure d'acide par du méthanol pendant 30 min, ce qui produit du méthoxyacétate de méthyle de basse température d'ébullition (129-130°C). On évapore les solvants et on reprend le résidu par du chloroforme, puis on le lave par une phase aqueuse à 5% de bicarbonate de sodium. On sèche la phase chloroformique sur du sulfate de sodium, puis on l'évapore, ce qui conduit à l'obtention d'un résidu orangé qui correspond au dérivé triprotégé.

On purifie le dérivé par chromatographie sur colonne de silice (diamètre 4 cm, longueur 10 cm) en utilisant un gradient chloroforme-méthanol, On recueille ainsi 7 g de dérivé triprotégé, ce qui correspond à un rendement de 73%.

On hydrolyse alors les fonctions ester au moyen d'un mélange de triéthylamine, de pyridine et d'eau (20/20/60 en volume). On évapore ensuite les solvants et on purifie la N₂-méthoxyacétyl désoxy-2' guanosine (composé n°3) par chromatographie sur colonne de silice silanisée en réalisant l'élution par un mélange d'eau et d'acétone (70/30 v/v). On obtient ainsi 3,4 g de produit, ce qui correspond à un rendement de 51%. On contrôle le produit par résonance magnétique nucléaire à 250 MHz et par spectrométrie de masse et l'on obtient les résultats suivants :
- (M - H) : pic moléculaire (m/e : 338 - 10%) ; guanine méthoxy-acétylée : (m/e : 222 - 31%).

### EXEMPLE 4 : Préparation de (N₆-phénoxyacétyl)-désoxy-2' adénosine (composé n°4)

On sèche 1025 mg (4 mmol) de désoxyadénosine puis on les dissout dans 20 ml de pyridine distillée anhydre et on les introduit dans un ballon placé dans un bain d'eau glacée. On ajoute alors lentement 8 équivalents d'anhydride phénoxyacétique (9,5 g ; 32 mmol) dissous dans 20 ml de pyridine à 0°C. On laisse la réaction se poursuivre à la température ambiante pendant 90 min et une coloration jaunâtre apparaît progressivement. On forme ainsi le dérivé triprotégé de nucléoside de départ.

On détruit alors l'excès d'anhydride d'acide à 0°C par addition de 3 ml d'eau distillée, puis on dilue le milieu réactionnel par 100 ml de chloroforme. On lave la phase chloroformique 4 fois au moyen de 50 ml d'une solution aqueuse à 5% de bicarbonate de sodium, et on évapore le solvant, ce ml conduit à l'obtention d'un résidu jaune. On dissout celui-ci dans 100 ml de pyridine et, après avoir placé la solution dans un bain d'eau glacée, on ajoute 100 ml de soude 0,2 N à 0°C pour hydrolyser sélectivement les positions 3' et 5' de l'adénosine en 15 min. On neutralise alors la résine échangeuse de cations Dowex 50W-X8 sous forme pyridinium utilisée dans l'exemple 1. On filtre et on rince la résine puis on évapore à sec le filtrat.

On obtient ainsi la (N₆-phénoxyacétyl)-désoxy-2' adénosine (composé n°4) que l'on purifie par chromatographie sur colonne de silice (diamètre 4 cm et longueur 10 cm) en utilisant un gradient de chloroforme-méthanol (100-0-96-4). On évapore alors les fractions contenant le produit recherché et l'on obtient ainsi 1010 mg d'une poudre blanchâtre, ce qui correspond un rendement de 65%.

On caractérise alors le produit par chromatographie sur couche mince, par résonance magnétique nucléaire du proton à 250 MHz et par spectrométrie de masse. Les résultats Obtenus sont les suivants :
- R_{F} : 0,66 avec le mélange de migration chloroforme-méthanol (80/20 en volume),
- résonance magnétique nucléaire du proton à 250 MHz : ¹H-RMN (pyridine d₅) : 2,7-3,3 (m, 2H, H₂, H_{2"}) ; 4,1-4,35 (m, 2H, H₅H_{5"}) ; 4,6 (m, H_{4'}) ; 5,25 (m, H_{3'}) 5,65 (s, 2H, CH₂) ; 7,0 (m, H_{1'}) 6,9-7,4 (m, 5H, C₆H₅) 8,75 et 9,05 (s, H₂ et H₈).
- spectrométrie de masse : (M+H) : pic moléculaire (m/e : 386-16%) ; adénine phénoxy acétylée (m/e : 270 - 66%).

### EXEMPLE 5 : Préparation de (N₄-isobutyryl)-désoxy-2' cytidine (composé n°5)

On sèche 4 mmol de désoxycytidine puis on les dissout dans 15 ml de pyridine anhydre et on introduit la solution dans un ballon placé dans un bain d'eau glacée. On ajoute alors lentement 6 équivalents (2,5 ml ; 24 mmol) de chlorure d'isobutyryle. On laisse la réaction se poursuivre à la température ambiante pendant 120 min et le milieu prend une teinte orangée. On forme ainsi le dérivé triprotégé du nucléoside de départ, soit Le dérivé de formule (I) dans laquelle, R³ et R⁴ représentent le radical isobutyryle et R⁵ représente un atome d'hydrogène.

On détruit l'excès de chlorure d'acide à 0°C par 2 ml d'eau bidistillée et on dilue le milieu réactionnel par 100 ml de chloroforme. On lave la phase chloroformique 4 fois par 50 ml de solution à 5% de bicarbonate de sodium pour éliminer l'acide isobutyrique formé, on sèche sur du sulfate de sodium et on évapore à sec. On obtient un résidu orange que l'on dissout dans 5 ml de pyridine refroidie à 0°C. On ajoute alors 100 ml de soude 0,2 N à 0°C et on laisse la réaction d'hydrolyse sélective des fonctions ester en 3' et 5' du désoxyribose se poursuivre pendant 30 min. On neutralise alors le milieu par la résine échangeuse d'ions Dowex 50W-X8 sous forme pyridinium utilisée dans l'exemple 1. On filtre ensuite et on rince la résine, puis on évapore à sec le filtrat.

On purifie par chromatographie sur colonne de silice (diamètre 4 cm ; L : 10 cm) en utilisant un gradient de chloroforme-méthanol (100-0 à 95-5). On évapore les solvants et l'on obtient ainsi 630 mg d'une poudre blanche constituée d'isobutyryl-désoxycytidine, ce qui correspond à un rendement de 50%.

On caractérise le produit par chromatographie sur couche mince, spectrométrie de masse et résonance magnétique nucléaire du proton à 250 MHz. Les résultats obtenus sont les suivants :
- R_{F} : 0,55 avec le mélange de migration chloroforme-méthanol (80/20),
- spectrométrie de masse (M+H) : pic moléculaire (m/e = 298-11%), cytosine isobutyrylée (m/e = 182-100%)
- résonance magnétique nucléaire du proton à 250 MHz : ¹H-RMN (méthanol d₄) : 1,2 (d, 6H, 2(CH₃) ) de ib.), 2,15-2,6 (m, 2H, H₂, H_{2"}), 2,7 (m, H^{ib}) ; 3,7-3,9 (m, 2H, H_{5'}, H_{5"}) ; 4,0 (m, H₄) ; 4,4 (m, H_{3'}) ; 6,25 (t, H_{1'}) ; 7,5 et 8,5 (d, H₅ et H₆).

### EXEMPLE 6 : Préparation de [N₄-isobutyryl)(diméthoxy-4,4'-trityl)-5' désoxy-2' cytidine (composé n°6)

On sèche 2,5 mmol du composé n° 5 par additions et évaporations successives de pyridine anhydre. On reprend par 25 ml de pyridine, on refroidit à 0°C et on ajoute 2,75 mmol (1,1 équivalent) de chlorure de diméthoxy-4,4' trityle dans 25 ml de pyridine, à 0°C. On laisse la réaction se poursuivre pendant 17 h à 5°C et on ajoute alors 2 ml de méthanol au milieu réactionnel. Après 30 min, on chasse le solvant à l'évaporateur rotatif et on reprend le résidu huileux par 100 ml d'acétate d'éthyle, et on lave par 3 fois 50 ml de solution aqueuse à 5% de NaHCO₃ et une fois 50 ml d'eau bidistillée. On sèche alors la phase organique sur sulfate de sodium et on la concentre. Par fractionnements sur colonne de gel de silice, on isole le composé n°6 ainsi obtenu qui répond à la formule (I) dans laquelle , R³ est le radical diméthoxy-4,4' trityle, R⁴ est un atome d'hydrogène et R⁵ est un atome d hydrogène.

Les caractéristiques physico-chimiques de ce composé et le rendement de la réaction sont donnés dans le tableau 1 annexé.

### EXEMPLE 7 : préparation de (N₆-phénoxyacétyl)-(diméthoxy-4,4' trityl)-5' désoxy-2' adénosine (composé n°7)

On suit le même mode opératoire que dans l'exemple 6, mais en utilisant 2,5 mmol du composé n°4 au lieu du composé n°5 et l'on produit ainsi le composé n°7 : (N₆-phénoxyacétyl)(diméthoxy-4,4' trityl)-5' désoxy-2' adénosine.

Le rendement de la réaction et les caractéristiques physico-chimiques de ce composé sont données dans le tableau I annexé.

### EXEMPLE 8 : préparation de (N₂-méthoxyacétyl)-(diméthoxy-4,4' trityl)-5' désoxy-2' guanosine (composé n°8)

On suit le même mode opératoire que dans l'exemple 6, mais en utilisant 2,5 mmol du composé n°3 au lieu du composé n°5. On obtient ainsi le composé n°8.

Le rendement de la réaction et les caractéristiques physico-chimiques de ce composé sont donnés dans le tableau 1 annexé.

Dans ce tableau, les lettres entre parenthèses indiquent la multiplicité du pic avec s=singulet, d=doublet, t=triplet ; q=quadruplet et m=multiplet.

### EXEMPLE 9 : Préparation du composé n°9

Cet exemple 1 llustre la préparation d'un dérivé phosphorylé du composé n°8 destiné à la synthèse phosphotriester de polynucléotides.

On sèche 3 mmol du composé n°8 par ajout et évaporation de pyridine anhydre (3 fois 5 ml). On reprend le résidu dans 15 ml de pyridine et on ajoute 4,5 mmol de bistriazolidate de chloro-4 phénylphosphoryle dans 30 ml de dioxanne anhydre (le bistriazolidate de chloro-4 phénylphosphoryle a été obtenu en ajoutant 4,5 mmol de dichlorophosphate de chloro-4 phényle à une suspension de 9 mmol de triazole et 9,35 mmol de triéthylamine dans 30 ml de dioxanne). On laisse la réaction se poursuivre pendant 20 min et on la stoppe alors par addition de 6 ml d'un méLange H₂O-triéthylamine (1/1 en volume) puis on réduit le volume du milieu réactionnel à 5 ml par évaporation. On reprend dans 100 ml de chloroforme et on Lave 3 fois avec 50 ml de solution aqueuse de NaHCO₃ puis avec 100 ml d'eau. On sèche la phase chloroformique sur sulfate de sodium, puis on l'évapore à sec. Le composé recherché est isolé par chromatographie sur gel de silice. On contrôle alors le produit obtenu par spectrométrie de masse et par résonance magnétique nucléaire. Le rendement de la réaction et les résultats obtenus sont donnés dans le tableau 2 annexé.

Le composé n°9 ainsi obtenu répond à la formule annexée.

### EXEMPLE 10 : Préparation du composé n°10

Dans cet exemple, on suit le même mode opératoire que dans l'exemple 9 pour préparer le dérivé phosphorylé destiné à la synthèse phosphotriester du composé n°7 en utilisant 2,5 mmol du composé n°7 au lieu de 2,5 mmol du composé n°8. On obtient ainsi le composé n°10 dont la formule est donnée en annexe.

On contrôle comme précédemment les caractéristiques du composé obtenu par spectrométrie de masse et par résonance magnétique, les résultats obtenus et le rendement de la réaction sont donnés également dans le tableau 2 annexé.

### EXEMPLE 11 : Préparation de composé n°11

Dans cet exemple, on suit le même mode opératoire que dans l'exempte 9 pour préparer le dérivé phosphorylé du composé n°6 destiné à la synthèse phosphotriester en utilisant 3 mmol du composé n°6 au lieu de 3 mmol du composé n°8.

On obtient ainsi le composé n°11 dont la formule est donnée en annexe.

Comme dans les exemples 9 et 10, on contrôle le produit obtenu par spectrométrie de masse et par résonance magnétique nucléaire. Le rendement de la réaction et les résultats obtenue sont donnés dans le tableau 2 annexé.

### EXEMPLE 13 : Préparation du composé n°12

Dans cet exemple, on prépare le dérivé phosphorylé du composé n°8 destiné à la synthèse phosphoramidite d'otigonuctéotides.

On sèche 3 mmol de composé n°8 par co-évaporation de pyridine, de toluène et de tétrahydrofuranne (THF). On reprend le résidu dans 15 ml de THF en présence de 12 mmol de N,N,N-diisopropyléthylamine et on ajoute goutte à goutte en 2 min 6 mmol de -cyanoéthyl-monochloro N,N-diisopropylaminophosphoramidite. Après 5 min de réaction, on observe la formation d'un précipité de chlorhydrate de l'amine. On laisse la réaction se poursuivre pendant 35 min et on filtre le précipité en fin de réaction, puis on évapore à sec le filtrat et on le reprend dans 150 ml d'acétate d'éthyle. On lave par une solution aqueuse glacée à 10% de Na₂CO₃. On sèche ensuite la phase organique sur sulfate de sodium et on l'évapore à sec.

On purifie le composé obtenu par chromatographie basse pression sur une colonne MERCK "LOBAR" de taille B en utilisant pour l'élution un mélange CH₂Cl₂-hexane-triéthylamine (70/20/10 en volume). On reprend le composé obtenu par un minimum de dichlorométhane ou d'acétate d'éthyle et on le précipite dans l'hexane à -80°C. On analyse le produit par résonance magnétique nucléaire. Les résultats obtenus et le rendement de la réaction sont donnés dans le tableau 3 annexé.

On obtient ainsi le composé n°12 dont la formule est donnée en annexe.

### EXEMPLE 13 : Préparation du composé n°13

Dans cet exemple, on prépare comme dans l'exemple 12 le dérivé phosphorylé du composé n°7 destiné à la synthèse phosphoramidite en utilisant 3 mmol du composé n°7 au lieu de 3 mmol du composé n°8. On analyse également le produit obtenu par résonance magnétique nucléaire. Le rendement de la réaction et les résultats sont donnés dans le tableau 3 annexé.

On obtient ainsi le composé n°13 dont la formule est donnée en annexe.

### EXEMPLE 14 : Préparation du composé N°14

Dans cet exemple, on prépare comme dans l'exemple 12 le dérivé phosphorylé du composé n°6 destiné à la synthèse phosphoramidite en utilisant 3 mmol du composé n°6 au lieu de 3 mmol du composé n°8. On analyse également le produit obtenu par résonance magnétique nucléaire. Le rendement de la réaction et les résultats obtenus sont donnés dans le tableau 3 annexé.

On obtient ainsi le composé n°14 dont la formule est donnée en annexe.

### EXEMPLE 15 : Préparation de la N6-phénoxyacétyl (diméthoxy-4,4' trityl)-5' désoxy-2' guanosine (composé n°15).

On suit le même mode opératoire que dans l'exemple 6, en utilisant 4 mmol du composé n°1 au lieu du composé n°5 et l'on produit ainsi le composé n°15.

Le rendement de la réaction est de 70%. Le Rf de ce composé dans le mélange Chloroforme/Méthanol (90:10) vaut 0,40. Les déplacements chimiques des principaux protons de cette molécule dans le méthanol deutérié ont les valeurs suivantes : H8 : 8,07 ppm, H1' : 6,45 ppm (t), H3' : 4,75 ppm (m), H CH3 de diméthoxy trityle : 3,86 ppm (s), H CH2 de phénoxyacétyle : 5,05 ppm (s).

### EXEMPLE 16 : Préparation du composé n°16.

Dans cet exemple on prépare comme dans l'exemple 12 le dérivé phosphorylé du composé n°15, destiné à la synthèse phosphoramidite en utilisant 3 mmol du composé n°15 au lieu de 3 mmol du composé n°8. Le rendement en produit final est de 50%. Le produit est caractérisé par un doublet en RMN du 31P localisé à 146 et 146,2 ppm dans la pyridine deutériée. Les principaux pics en RMN du proton dans l'acétonitrile deutérié sont localisés à : 8,12 ppm (s, H8), 6,45 ppm (t, H1'), 5,05 ppm (s, CH2 phénoxyacétyle) et 4,88 ppm (m, H3'). Par spectrométrie de masse à l'aide d'une source d'ions FAB il est possible d'observer le pic moléculaire de ce produit pour une valeur m/e de 903.

On obtient ainsi le composé n°16.

Les exemples 17 à 19 qui suivent illustrent la préparation de mononucléotides complètement protégés destinés à la synthèse d'oligonucléotides selon la méthode H-phosphonate.

### EXEMPLE 17 : Préparation du composé n°17.

Dans cet exemple, on prépare le composé n°17 destiné à la synthèse des oligonucléotides selon la méthode H-Phosphonate.

On sèche 5 mmol de composé n°7 par co-évaporation de 5 ml de dioxanne anhydre et on les reprend dans 15 ml de ce solvant et 5 ml de pyridine anhydre. On additionne alors 5 ml d'une solution 1,25 de chloro-2 benzo-5,6-a) [dioxo-1,3 phosphor-2 inone-4] salicylchlorophosphite de formule 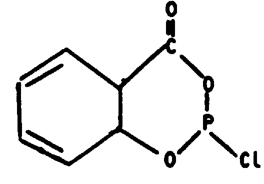 et on laisse la réaction se dérouler pendant 10 minutes à la température ambiante.

On additionne alors 0,5 ml d'eau et on laisse évoluer l'hydrolyse pendant 10 minutes. On verse alors ce mélange dans 250 ml de solution aqueuse molaire d'acétate de triéthylammonium et l'on extrait le produit désiré avec 2 fois 250 ml de chloroforme. On sèche la phase organique avec du sulfate de sodium anhydre et on la concentre à l'évaporateur rotatif. On purifie le résidu ainsi obtenu par chromatographie liquide haute performance sur une colonne de gel de silice (200x40 mm). On élue le produit avec des solutions à concentration croissante de méthanol dans un mélange à 2% de triéthylamine dans le chloroforme (0% de méthanol : 250 ml ; 1% de méthanol : 250 ml ; 2% de méthanol : 250 ml ; 3% de méthannol : 250 ml ; 5% de méthanol : 250 ml ; 7% de méthanol : 500 ml). On rassemble les fractions contenant le produit recherché et on évapore le solvant pour obtenir un produit se présentant sous la forme d'une mousse blanche. Le rendement en produit N°17 est de 55%. on analyse le produit par résonance magnétique nucléaire, les résultats obtenus sont regroupés dans le tabeau IV.

On obtient ainsi le composé n°17 dont la formule est donnée en annexe.

### EXEMPLE 18 : Préparation du composé n°18.

Dans cet exemple, on prépare comme dans l'exemple 17 le dérivé phosphorylé du coeposé n°15 destiné à la synthèse d'oligonucléotides selon la méthode H-phosphonate en utilisant 5 mmol du composé n°15 au lieu de 5 mmol du composé n°7. Le rendement de la réaction est de 48% et on analyse le produit obtenu par résonance magnétique nucléaire. Les résultats obtenus sont présentés dans le tableau IV.

On obtient ainsi le composé n°18 dont la formule est donnée en annexe.

### EXEMPLE 19 : Préparation du composé n°19.

Dans cet exemple, on prépare comme dans l'exemple 17 le dérivé phosphorylé du composé n°5 destiné à la synthèse d'oligonucléotides selon la méthode H-phosphonate en utilisant 5 mmol du composé n°5 au lieu de 5 mmol du composé n°7.

Le rendement de la réaction est de 62% et on analyse le produit obtenu par résonance magnétique nucléaire. Les résultats obtenus sont présentés dans le tableau IV.

On obtient ainsi le composé n°19 dont la formule est donnée en annexe.

### EXEMPLE 20 :

Cet exemple illustre l'utilisation des composés n° 9, n°10 et n°11 pour la synthèse d'un oligonucléotide dont la séquence est la suivante :

d(AATTCAGATUTGATCAT) AGRE-AGRE

Dans cette séquence, A représente le nucléotide formé avec l'adénine, C représente le nucléotide formé avec la cytosine et G représente le nucléotide formé avec la guanine, T représente le nucléotide formé avec la thymine et U le nucléotide formé avec l'uracile.

Pour effectuer cette synthèse, on utilise les composés n°9, n°10 et n°11 comme synthons correspondant respectivement à la guanine, à l'adénine, et à la cytosine et des synthons correspondant à la thymine et à l'uracile. Ceux-ci sont obtenus à partir des nucléosides correspondants en protégeant les fonctions hydroxyle en 5' et en 3' respectivement par le radical diméthoxy4,4' trityle et le radical de formule VI) en utilisant le même mode opératoire que dans les exemples 6 à 11.

On effectue la synthèse au moyen d'un automate Biosearch "SAM ONE" en utilisant :
- 50 mg, soit environ 1,5 à 3 mol, du support comportant l'extrémité 5' de la chaîne (Pierce "Controlled Pore Glass"),
- 25 mg par cycle de condensation des dérivés de nucléosides obtenus dans les exemples 9 à 11 et des synthons de thymidine et de désoxy-2' uridine, ce qui représente environ 8 à 15 équivalents, et
- 25 mg par cycle de condensation, soit 2 équivalents par rapport au nucléoside, d'un agent d'activation constitué par du chlorure de mésitylène sulfonyle.

Les étapes composant chaque cycle de condensation sont les suivantes :
- détritylation : acide trichloracétique à 2 % dans CH₂Cl₂ pendant 2 min,
- lavage : CH₃CN pendant 1 min,
- séchage : CH₃CN anhydre pendant 6 min,
- condensation : dérivé de nucléoside monomère et chlorure de mésitylène sulfonyle dans le mélange CH₃CN/méthyl-1 imidazole (85/15 en volume pendant 15 min, et
- lavage : CH₃CN pendant 6 min.

A la fin des cycles de condensation, on obtient donc un gel de silice comportant l'oligonucléotide lié de façon covalente. On transfère alors le tout dans une ampoule en pyrex, on ajoute de l'ammoniaque à 28%, et on la laisse pendant 8 h à la température ambiante. On libère ainsi l'oligonucléotide du support en éliminant également les groupes protecteurs faisant l'objet de l'invention.

On prélève alors le surnageant et on rince la silice avec 3 fois 1 ml d'eau bidistillée. On évapore le solvant et on reprend le résidu dans 0,5 ml d'eau, puis on le fractionne sur une colonne de Séphadex G 25 (diamètre 1 cm, H 7 cm), on recueille les fractions qui présentent une absorption dans l'ultraviolet à 254 nm et on les lyophilise.

On vérifie par marquage au phosphore 32 au moyen de la T4-polynucléotide kinase et par électrophorèse sur gel de polyacrylamide la bonne longueur du fragment d'ADN synthétique obtenu. Le découpage de la bande correspondante et l'élution du composé permettent de purifier le produit en vue d'une utilisation biologique.

On prépare de la même façon les oligonucléotides dont les séquences sont les suivantes :
- d(AATTCAGAUCTGATCAT),
- d(AATTCAGUTCTGATCAT),
- d(AATTCAUATCTGATCAT), et
- d(CGATGATCAGATCTG).

On obtient également de bons résultats et une élimination des groupes protecteurs dans des conditions douces, dans l'ammoniaque à température ordinaire.

### EXEMPLE 21

Dans cet exemple, on utilise les composés n°12, n°13 et n°14 pour synthétiser des homopolymères de 15 nucléotides de longueur en utilisant la synthèse phosphoramidite.

On réalise les synthèses à l'aide du même automate Biosearch "SAM ONE" que dans l'exemple 20 et on utilise les quantités de réactif suivantes :
- 50 mg, soit de 1,5 à 3 mol du support utilisé dans l'exemple 15 comportant l'extrémité 3' de la chaîne polynucléotidique,
- 20 mg des composés 12, 13, ou 14 par cycle de condensation (20 à 25 équivalents), et
- 15 mg par cycle de condensation (2 équivalents par rapport au nucléotide) d'un agent d'activation constitué par le paranitrophényl-5 tétrazole.

Chaque cycle de condensation comporte les étapes suivantes :
- détritylation : acide trichloracétique à 2 % dans CH₂Cl₂ pendant 1 min 30,
- lavage : CH₃CN pendant 1 min,
- séchage : CH₃CN anhydre/diméthylformamide anhydre (90/10) pendant 3 min,
- condensation : dérivé de nucléoside + agent d'activation dans le mélange CH₃CN/diméthylformamide (90/10) pendant 3 min,
- oxydation : iode à 0,45% dans tétrahydrofuranneleau/lutidine (89,5/10/0,5) pendant 1 min,
- masquage des fonctions hydroxyle n'ayant pas réagi pour stopper l'élongation des chaînes incomplètes mélange d'anhydride acétique et de méthyl-1 imidazole dans CH₃CN anhydre pendant 2 min,
- lavage : CH₃CN pendant 3 min.

Après 14 cycles de condensation réalisés avec le même composé (composés n° 12, n°13 ou n°14), on transfère le support comportant le produit synthétisé dans une ampoule de pyrex et on ajoute 2 ml d'ammoniaque à 28%. On maintient les ampoules pendant 8 h à la température ambiante, ce qui permet d'éliminer les groupes protecteurs faisant l'objet de l'invention.

On prélève alors le surnageant et on rince la silice avec 3 fois 1 ml d'eau bidistillée, puis on chasse le solvant à l'évaporateur rotatif. On reprend le résidu brut dans 0,5 ml d'eau et on le purifie par chromatographie sur gel de Séphadex G 25. On recueille les fractions absorbant à 254 nm et on analyse leur contenu par électrophorèse sur gel de polyacrylamide après marquage au phosphore 32 par T4-polynucléotide kinase.

On contrôle ainsi que les 3 homopolymères synthétisés ont la longueur voulue et qu'ils correspondent respectivement à d(A₁₅), d(C₁₅) et d(G₁₅).

### EXEMPLE 22 : Préparation d'un oligonucléotide par la méthode H-phosphonate.

Cet exemple illustre l'utilisation des composés n°17, n°18 et n°19 pour la synthèse d'un oligonucléotide dont la séquence est la suivante :

5' d(ATGATCTACT) 3'

Dans cette séquence, A, G, C et T représentent les mêmes nucléotides que dans la séquence de l'exemple 20.

Pour effectuer l'assemblage, on utilise les composés n°17, n°18 et n°19 comme synthons correspondant respectivement à l'adénine, la guanine et la cytosine et un synthon correspondant à la thymine. Ce dernier est obtenu à partir de la thymidine en protégeant la fonction 5' par un groupement diméthoxy-4,4' trityle et la fonction 3' par le groupement phosphoré de formule 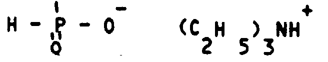 en utilisant le même mode opératoire que celui rapporté précédemment pour le dérivé H-phosphonate de l'adénine.

On effectue l'assemblage au moyen d'un automate Biosearch "SAM OHE" en utilisant :
- une colonne préconditionnée par le constructeur comprenant le support greffé avec 1 micromole de thymidine protégée en 5' par un groupement diméthoxy-4,4' trityle,
- 8 mg par cycle de condensation des dérivés de nucléosides n°17, n°18 et n°19 et du dérivé correspondant de la thymidine. Ce qui représente environ 10 équivalents molaires, et
- 6 microlitres par cycle de condensation soit 50 équivalents molaire de chlorure de triméthyl acétyle, utilisé comme agent d'activation.

Les étapes composant chaque cycle de condensation sont les suivantes :
- acide trichloracétique à 2% dans le dichlorométhane, 3ml en 1 minute,
- lavage à 1' acétonitrile : 3 ml,
- séchage avec un mélange pyridine/acétonitrile 1:1 / 3 ml,
- condensation : dérivé du nucléoside et agent d'activation dans 2 ml de mélange pyridine/acétonitrile en 1 minute,
- lavage par 3 ml de mélange de pyridine et d'acétonitrile,
- lavage par 3 ml d'acétonitrile.

A la fin des cycles de condensation, on procède à l'oxydation des phosphores internucléotidiques par passage d'une solution d'iode à 2% dans un mélange pyridine/eau 98/2 (3ml). On effectue ensuite un lavage par le mélange de pyridine et d'acétonirile (5ml), par l'acétonitrile (3ml) et l'on procède à la détritylation comme il est décrit ci-dessus.

On obtient alors un gel de silice comportant le polynucléotide lié de façon covalente. On transfère le tout dans une ampoule en pyrex, on ajoute 2ml d'ammoniaque à 28% et on la laisse pendant 2 heures à la température ordinaire. On libère ainsi l'oligonucléotide du support en éliminant également les groupements protecteurs faisant l'objet de l'invention. On prélève alors le surnageant, on rince la silice avec 3 fois 1 ml d'eau bidistillée, on évapore le solvant et on reprend le résidu par 0,5 ml d'eau. On le fractionne ensuite sur une colonne de Séphadex G25 (diamètre 1 cm, hauteur 7 cm), on réunit les fractions présentant une absorption dans l'ultraviolet à 254 nm et on les lyophilise.

On vérifie la bonne longueur du produit synthétisé par marquage radio-actif au phosphore 32 au moyen de la T4-polynucléotide kinase suivi d'une électrophorèse sur gel de polyacrylamide.

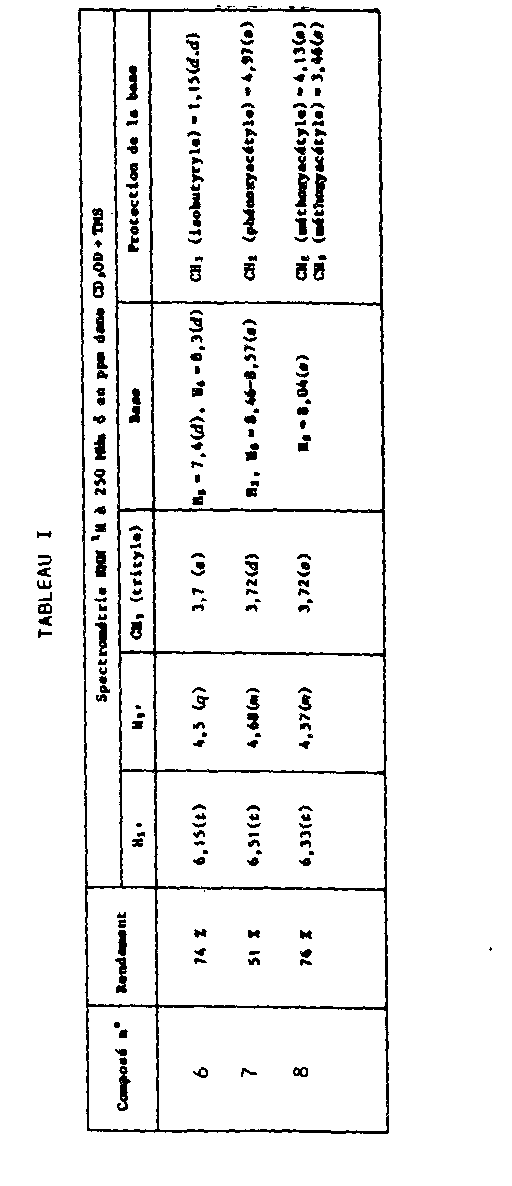

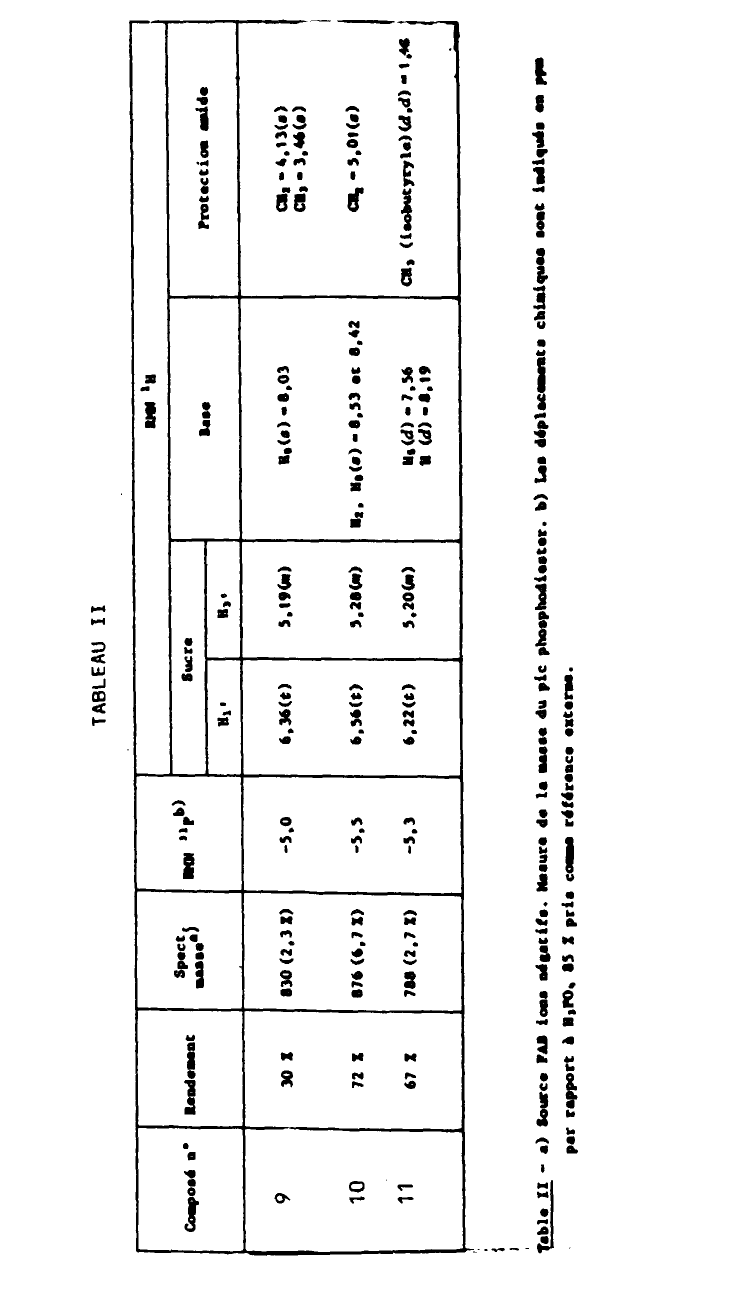

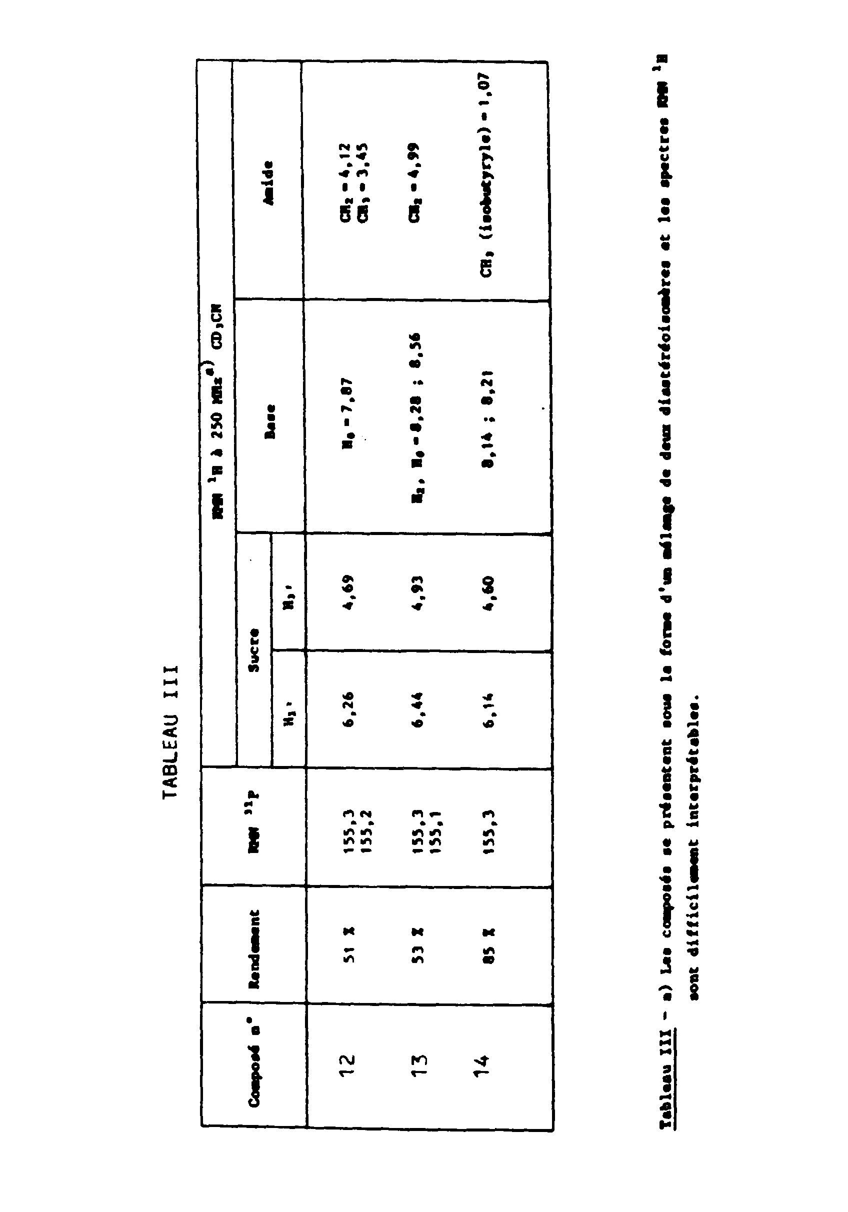

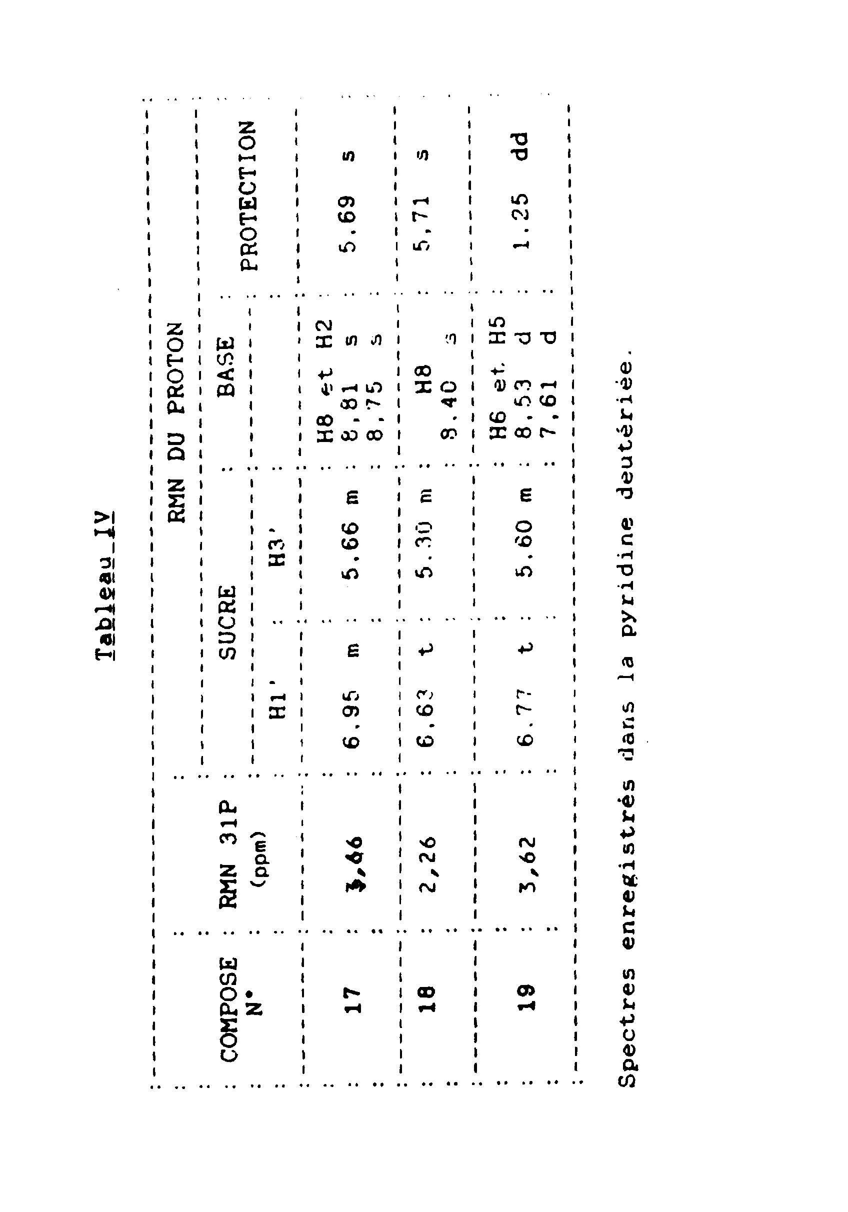

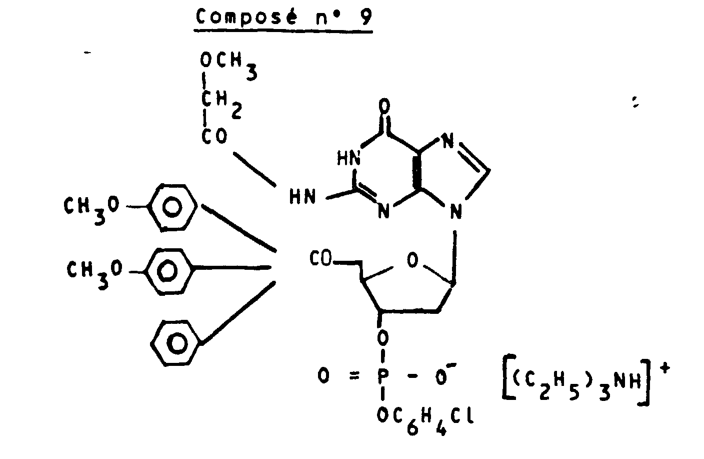

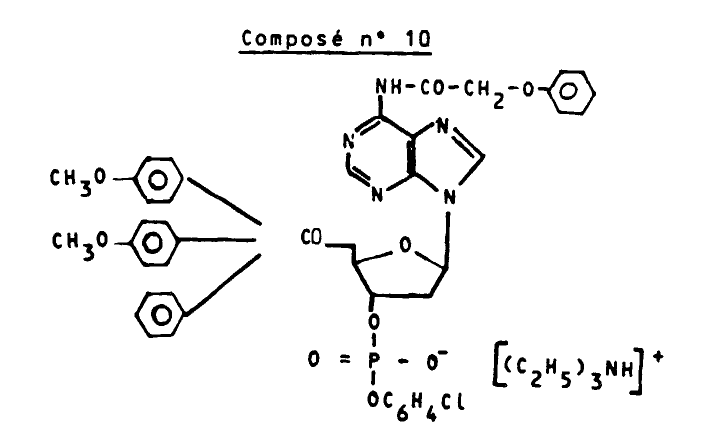

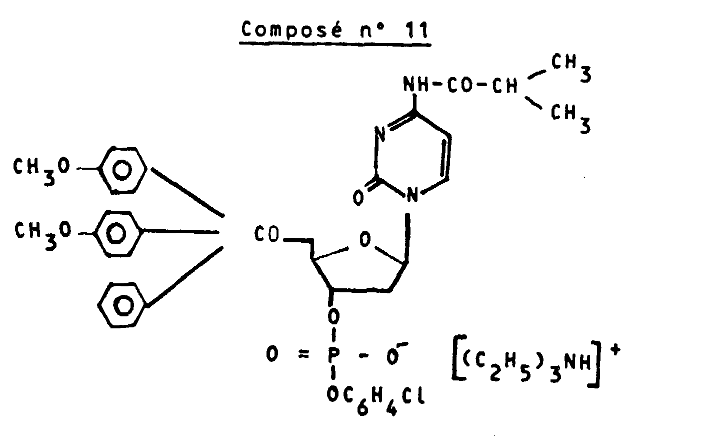

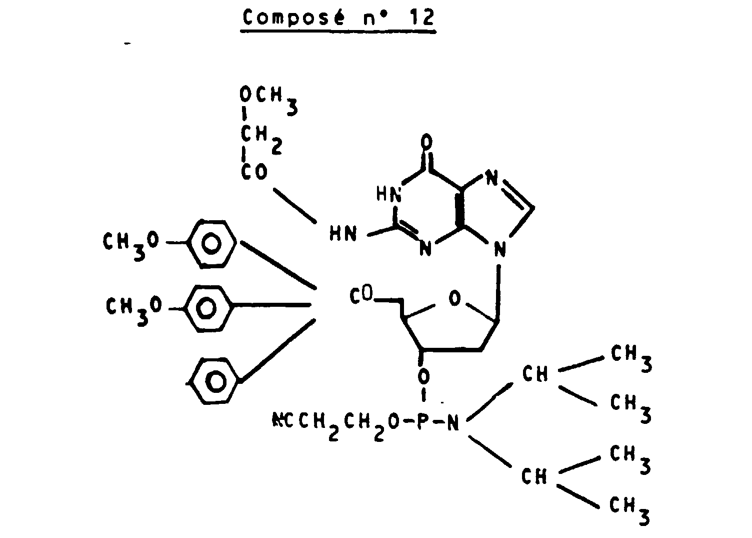

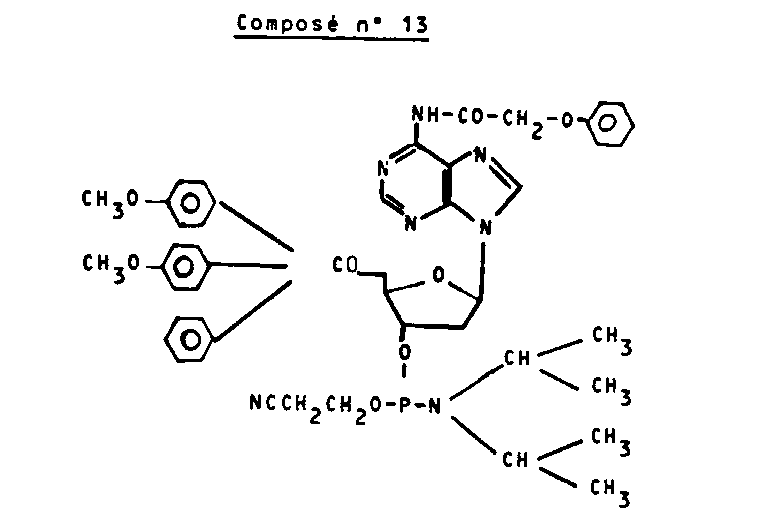

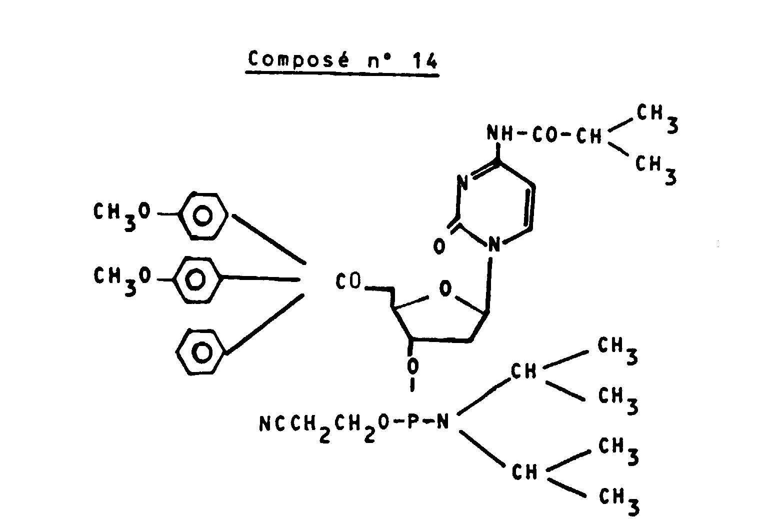

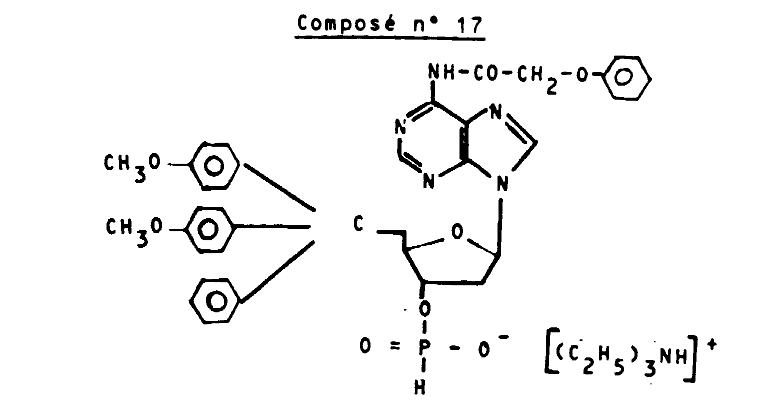

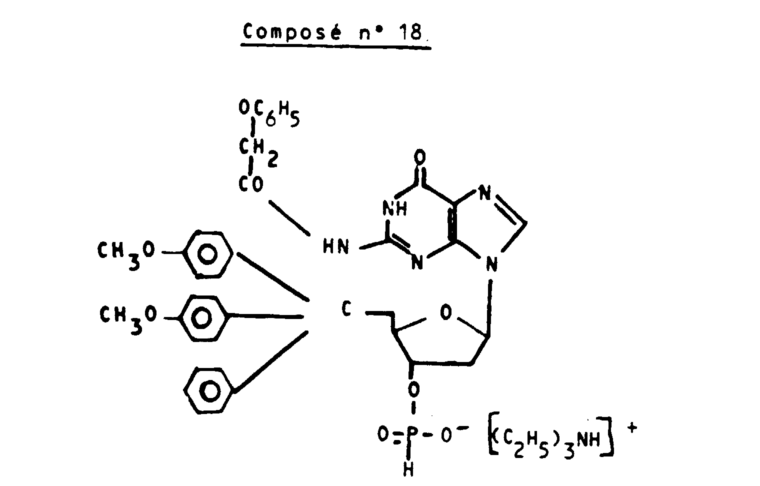

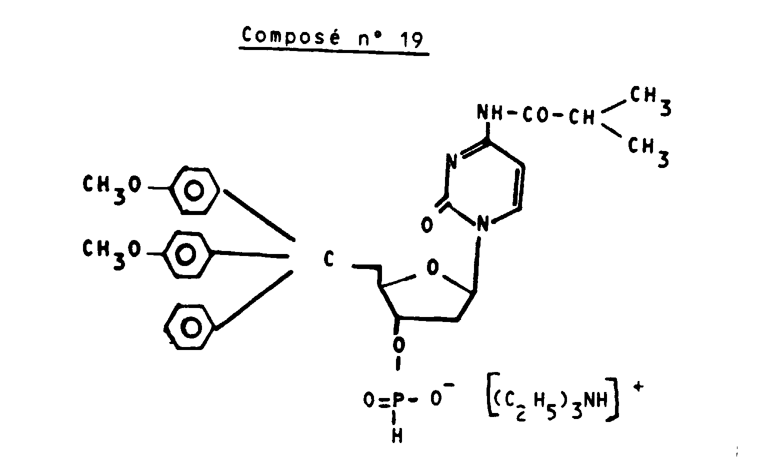

## Revendications

1. Dérivé de nucléoside répondant à la formule 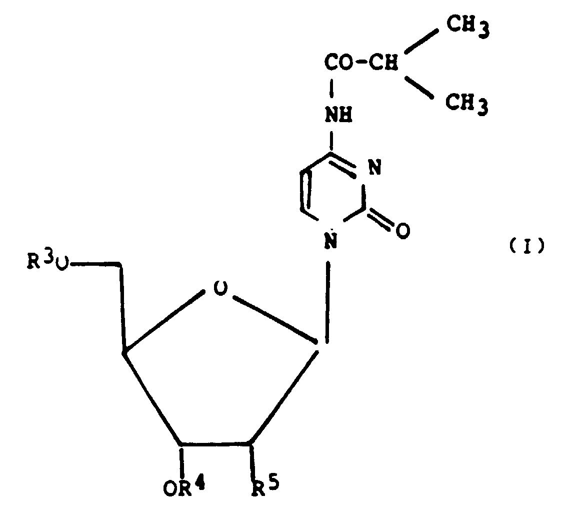 dans laquelle
- R³ représente un atome d'hydrogène, un radical labile en milieu acide utilisable pour la synthèse oligonucléotidique ou le radical de formule 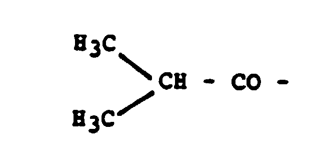
- R⁴ représente un atome d'hydrogène, un radical phosphoré utilisable pour la synthèse oligonucléotidique ou le radical : 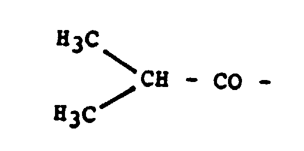 et
- R⁵ représente un atome d' hydrogène, et le radical OH ou un radical OH protégé.

2. Dérivé de nucléoside selon la revendication 1, caractérisé en ce que R³ est choisi dans le groupe des radicaux trityle de formule : 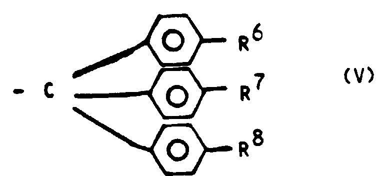 dans laquelle R⁶, R⁷ et R⁸ qui peuvent être identiques ou différents, représentent un atome d' hydrogène, un radical alkyle ou un radical alcoxy, et du radical phényl-9 xanthényle.

3. Dérivé de nucléoside selon l'une quelconque des revendications 1 et 2, caractérisé en ce que R⁴ est choisi dans le groupe des radicaux de formule : 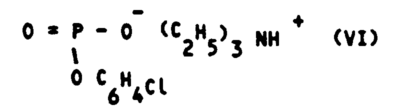 de formule : ou de formule :

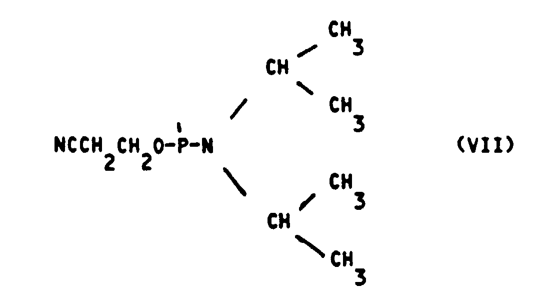

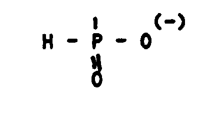

4. Dérivé de nucléoside selon l'une quelconque des revendications 1 à 3, caractérisé en ce que R³ et R⁴ représentent un atome d'hydrogène.

5. Dérivé de nucléoside selon la revendication 1, caractérisé en ce que R³ représente le radical : 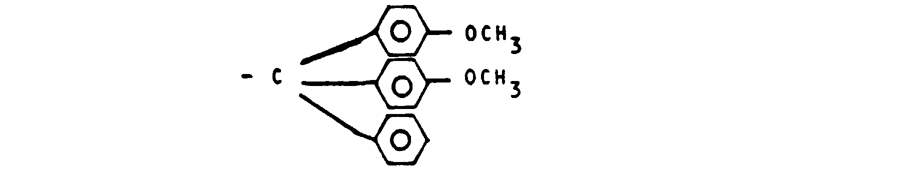 et R⁴ représente un atome d'hydrogène.

6. Dérivé de nucléoside selon l'une quelconque des revendications 1 à 5, caractérisés en ce que R⁵ représente un atome d'hydrogène.

7. Dérivé de nucléoside selon la revendication 1, caractérisé en ce que R³ représente le radical : 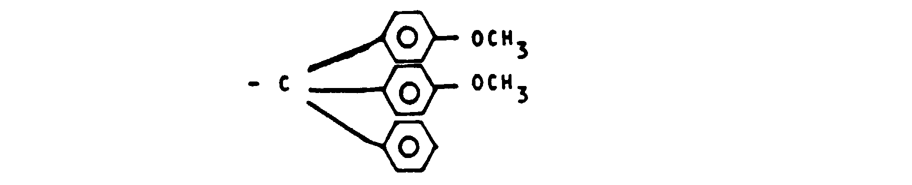 et R⁴ représente le radical :

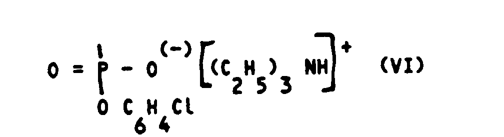

8. Dérivé de nucléoside selon la revendication 1, caractérisé en ce que R³ représente le radical : 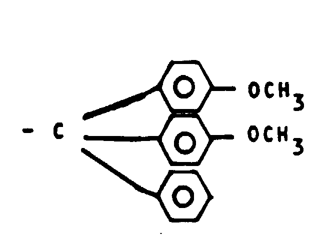 et R⁴ représente le radical :

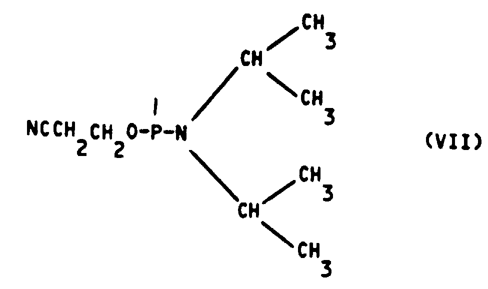

9. Dérivé de nucléoside selon la revendication 1, caractérisé en ce que R³ représente le radical : 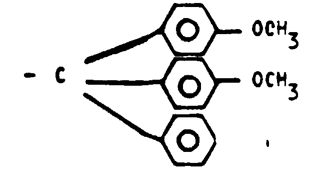 et R⁴ représente le radical de formule : 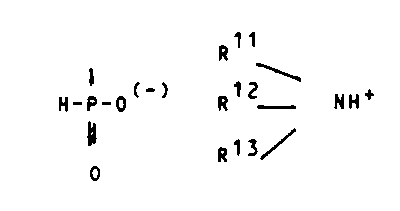 dans laquelle R¹¹, R¹² et R¹³ qui peuvent être identiques ou différents, sont des radicaux alkyle.

10. Dérivé de nucléoside selon l'une quelconque des revendications 7 à 9, caractérisé en ce que R⁵ représente un atome d' hydrogène.

11. procédé de synthèse d'oligonucléotides, caractérisé en ce qu'il comprend :
1°) au moins un cycle de condensation dans lequel on condense sur un dérivé de nucléoside ou sur un oligonucléotide un dérivé de nucléoside de formule : 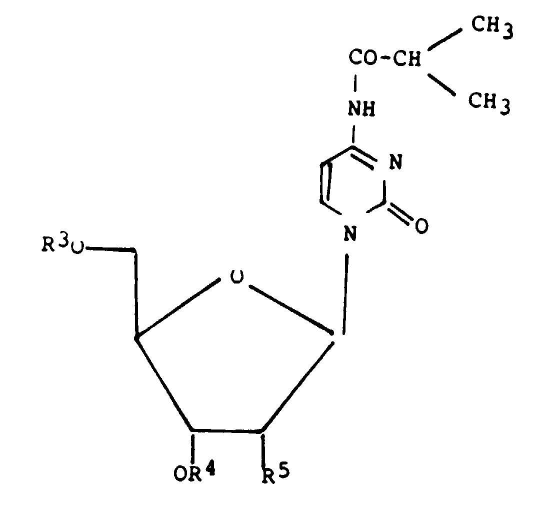 dans laquelle
- R³ représente un radical labile en milieu acide pour la synthèse oligonucléotidique,
- R⁴ représente un radical phosphoré pour la synthèse oligonucléotidique, et
- R⁵ représente un atome d' hydrogène ; et
2°) une étape d'élimination du ou des groupes protecteurs de formule :

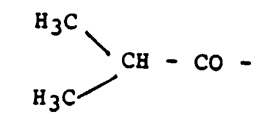

## Patentansprüche

1. Nucleosidderivat der Formel: 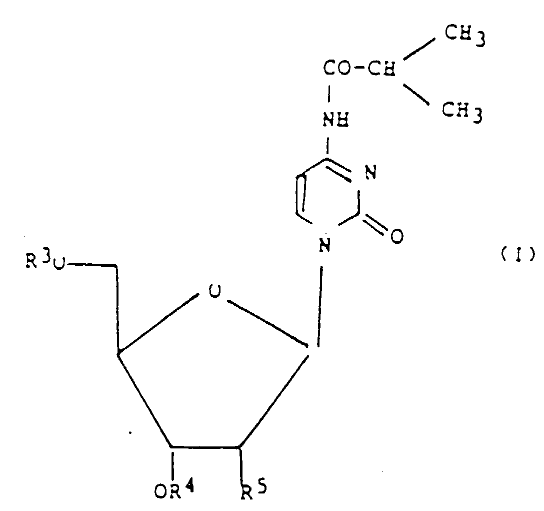 worin
R³ ein Wasserstoffatom, einen in saurer Umgebung labilen Rest, der zur oligonucleotid-Synthese verwendbar ist, oder den Rest der Formel: 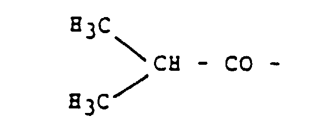 darstellt,
R⁴ ein Wasserstoffatom, einen phosphorhaltigen Rest, der zur oligonucleotid-Synthese verwendbar ist, oder den Rest: 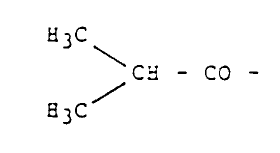 darstellt, und
R⁵ ein Wasserstoffatom und den Rest OH oder einen geschützten OH-Rest darstellt.

2. Nucleosidderivat nach Anspruch 1, **dadurch gekennzeichnet**, daß R³ ausgewählt ist aus der Gruppe der Tritylreste der Formel: 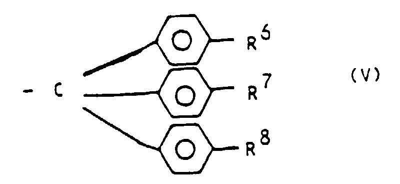 in welcher R⁶, R⁷ und R⁸, welche gleich oder verschieden sein können, ein Wasserstoffatom, einen Alkylrest oder einen Alkoxyrest darstellen, und dem 9-Phenyl-xanthenyl-Rest.

3. Nucleosidderivat nach einem der Ansprüche 1 und 2, **dadurch gekennzeichnet**, daß R⁴ gewählt ist aus der Gruppe der Reste der Formel: 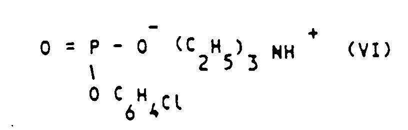 der Formel: 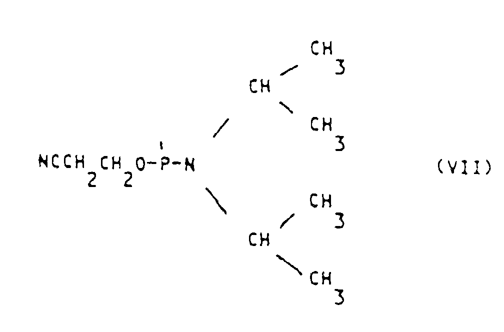 oder der Formel:

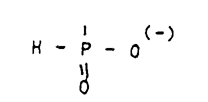

4. Nucleosidderivat nach einem der Ansprüche 1 bis 3, **dadurch gekennzeichnet**, das R³ und R⁴ ein Wasserstoffatom darstellen.

5. Nucleosidderivat nach Anspruch 1, **dadurch gekennzeichnet**, daß R³ den Rest: darstellt, und 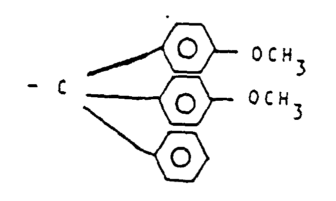 R⁴ ein Wasserstoffatom darstellt.

6. Nucleosidderivat nach einem der Ansprüche 1 bis 5, **dadurch gekennzeichnet**, das R⁵ ein Wasserstoffatom darstellt.

7. Nucleosidderivat nach Anspruch 1, **dadurch gekennzeichnet**, daß R³ den Rest: 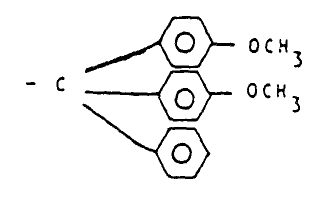 darstellt, und R⁴ den Rest: 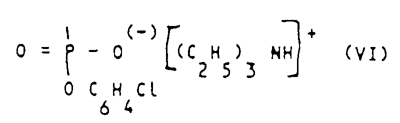 darstellt.

8. Nucleosidderivat nach Anspruch 1, **dadurch gekennzeichnet**, daß R³ den Rest: 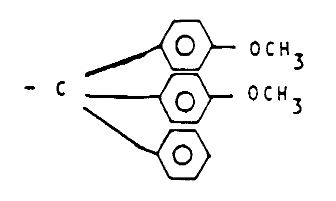 darstellt, und
R⁴ den Rest: 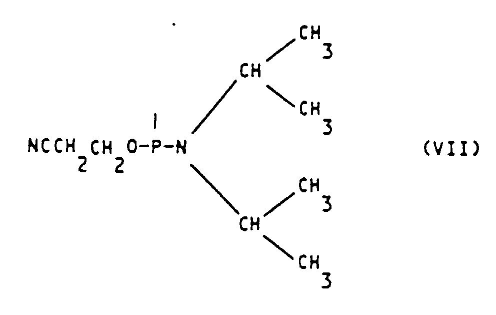 darstellt.

9. Nucleosidderivat nach Anspruch 1, **dadurch gekennzeichnet**, daß R³ den Rest: 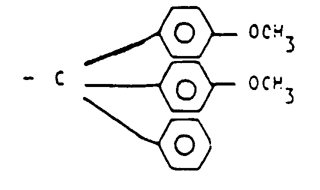 darstellt, und
R⁴ den Rest der Formel: 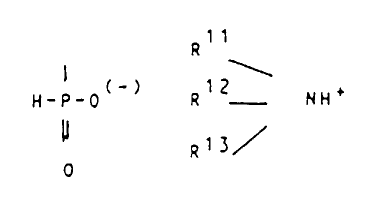 darstellt,
in welcher R¹¹, R¹² und R¹³, welche gleich oder verschieden sein können, Alkylreste sind.

10. Nucleosidderivat nach einem der Ansprüche 7 bis 9, **dadurch gekennzeichnet**, daß R⁵ ein Wasserstoffatom darstellt.

11. Verfahren zur Synthese von Oligonucleotiden, **dadurch gekennzeichnet**, daß es umfaßt:
1) mindestens einen Kondensationszyklus, in welchem an einem Nucleosidderivat oder an einem Oligonucleotid ein Nucleosidderivat der Formel: 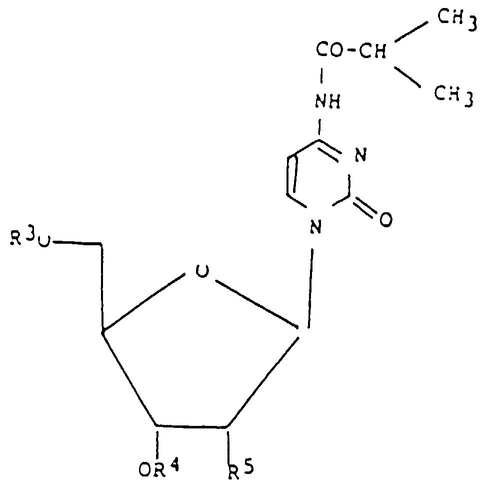 ankondensiert wird, in welcher
R³ einen in saurer Umgebung labilen Rest für die Oligonucleotid-Synthese darstellt,
R⁴ einen phosphorhaltigen Rest für die Oligonucleotid-Synthese darstellt und
R⁵ ein Wasserstoffatom darstellt; und
2) einen Schritt der Abspaltung der Schutzgruppe(n) der Formel:

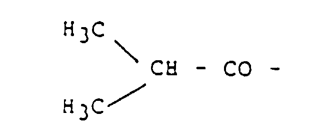

## Claims

1. Nucleoside derivative complying with formula 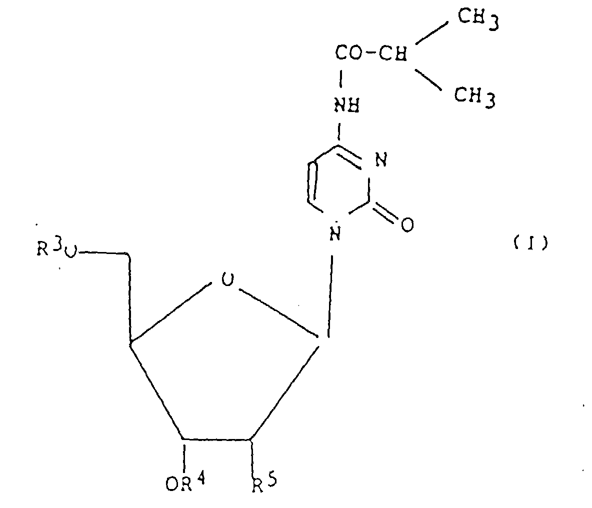 in which R³ represents a hydrogen atom, a radical unstable in an acid medium usable for oligonucleotide synthesis, or the radical of formula: 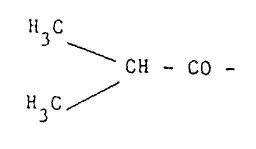
R⁴ represents a hydrogen atom, a phosphorus radical usable for oligonucleotide synthesis, or the radial: 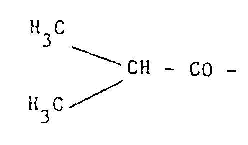 and
R⁵ represents a hydrogen atom and the OH radical or the protected OH radical.

2. Nucleoside derivative according to claim 1, characterized in that R³ is chosen from within the group of trityl radicals of formula: 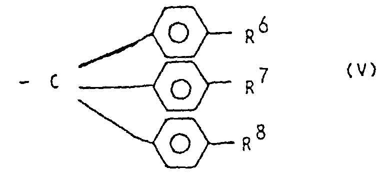 in which R⁶, R⁷ and R⁸, which can be the same or different, represent a hydrogen atom, an alkyl radical or an alkoxy radical, and the 9-phenyl xanthenyl radical.

3. Nucleoside derivative according to either of the claims 1 and 2, characterized in that R⁴ is chosen from within the group of radicals of formula: 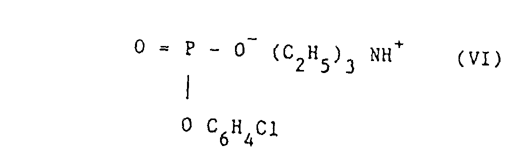 of formula: 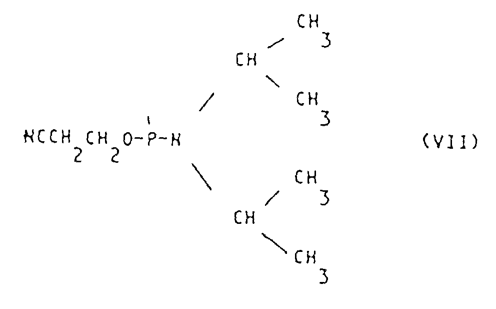 or of formula:

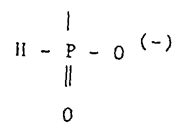

4. Nucleoside derivative according to any one of the claims 1 to 3, characterized in that R³ and R⁴ represent a hydrogen atom.

5. Nucleoside derivative according to claim 1, characterized in that R³ represents the radical: 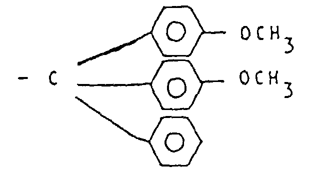 and R⁴ represents a hydrogen atom.

6. Nucleoside derivative according to any one of the claims 1 to 5, characterized in that R⁵ represents a hydrogen atom.

7. Nucleoside derivative according to claim 1, characterized in that R³ represents the radical: 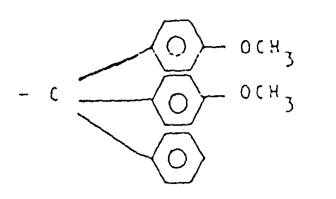 and R⁴ represents the radical:

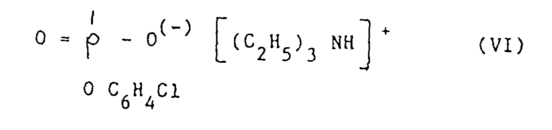

8. Nucleoside derivative according to claim 1, characterized in that R³ represents the radical: 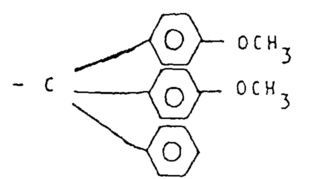 and R⁴ represents the radical:

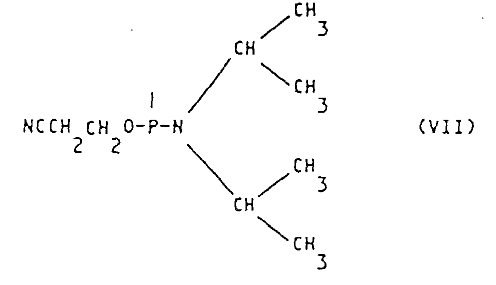

9. Nucleoside derivative according to claim 1, characterized in that R³ represents the radical: 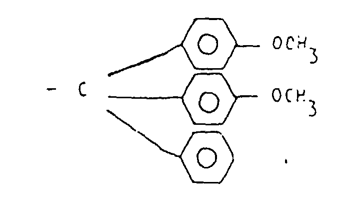 and R⁴ represents the radical of formula: 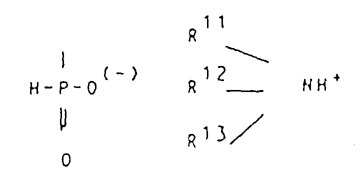 in which R¹¹, R¹² and R¹³, which can be the same or different, are alkyl radicals.

10. Nucleoside derivative according to any one of the claims 7 to 9, characterized in that R⁵ represents a hydrogen atom.

11. Process for the synthesis of oligonucleotides, characterized in that it comprises:
1) at least one condensation cycle, in which condensation takes place on a nucleoside derivative or on a nucleotide of a nucleoside derivative of formula: 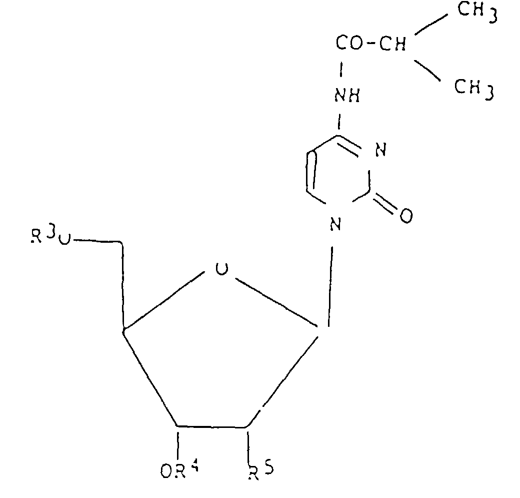 in which
- R³ represents a radical unstable in the acid medium for oligonucleotide synthesis,
- R⁴ represents a phosphorus radical for oligonucleotide synthesis and
- R⁵ represents a hydrogen atom and
2) a stage of eliminating the protecting group or groups of formula:
